# EUROPEAN PATENT APPLICATION

(11) **EP 1 792 916 A1**
(43) Date of publication of application: **06.06.2007**
(21) Application number: 06124703.7
(22) Date of filing: 24.11.2006
(51) Int. Cl.: C07K 14/72, C12N 15/12, A01K 67/027

(54) **TAAR1 transgenic animal**

(30) Priority: 05.12.2005 EP 05111699
(71) Applicant: F. HOFFMANN-LA ROCHE AG, 4070 Basel (CH)
(72) Inventor: Hoener, Marius, 4058 Basel (CH); Lindemann, Lothar, 4056 Basel (CH); Meyer, Claas Aiko, 4056 Basel (CH)

(57) **Abstract**

The present invention relates to genetic constructs comprising DNA encoding TAAR1 and their use to generate animals transgenic for TAAR1, transgenic animals comprising in their genome transgenic DNA encoding TAAR1, their use and methods of generating these animals.

## Description

The present invention provides genetic constructs comprising DNA encoding TAAR1 and methods for producing non-human transgenic animals comprising within their genome transgenic DNA encoding TAAR1. Furthermore, the present invention provides non-human transgenic animals comprising within their genomes transgenic DNA encoding TAAR1. Moreover, transgenic animals overexpressing TAAR1, as well as the methods of producing them are also provided. The invention also relates to the use of these animals as a tool for assessing TAAR1 function, for further characterization of known TAAR1 ligands and for identifying as yet unknown ligands of TAAR1, for analyzing TAAR1 signal transduction mechanisms, for analyzing the physiological function of TAAR1 *in vivo,* and for characterizing agonists, antagonists or modulators of TAAR1. Furthermore, the invention relates to the use of the said TAAR1 transgenic animals for studying the function of TAAR1 and their use as a model for identifying and testing the therapeutic effect of compounds regarding neurological, psychiatric and metabolic disorders.

Trace amines (TAs) are endogenous compounds related to biogenic amine neurotransmitters which are present in the mammalian nervous system in trace amounts. The intense research efforts on the pharmacology and metabolism of trace amines during the last decades has been triggered by their tight link to a variety of highly prevalent conditions such as depression, schizophrenia, anxiety disorders, bipolar disorder, attention deficit hyperactivity disorder, neurological diseases such as Parkinson Disease, epilepsy, migraine, hypertension, substance abuse and metabolic disorders such as eating disorders, diabetes, obesity and dyslipidemia (Reviewed in: Lindemann & Höner, 2005; Branchek, T.A. and Blackburn, T.P. (2003) Curr. Opin. Pharmacol. 3, 90-97; Premont, R.T., Gainetdinov, R.R., Caron, M.G. (2001) Proc. Natl. Acad. Sci. 98, 9474-9475.; Davenport, A.P. (2003) Curr. Opin. Pharmacol. 3,127-134.). However, a detailed understanding of the physiology of trace amines at the molecular level has only become possible with the recent identification of a novel family of G protein-coupled receptors termed Trace Amine Associated Receptors (TAARs; Lindemann, L., Ebeling, M., Kratochwil, N.A., Bunzow J.R., Grandy, D.K., Hoener, M.C. (2005) Genomics 85, 372-385; Bunzow, et al. (2001) Mol. Pharmacol. 60, 1181-1188; Borowsky, B., et al., (2001). Proc. Natl. Acad. Sci. U. S. A. 98, 8966-8971). Some of these receptors display sensitivity to trace amines, and their unique pharmacology and expression pattern make these receptors prime candidates for targets in drug development in the context of several diseases, some of which previously had been linked to trace amines. Progress in understanding the physiological relevance of TAARs and their ligands on the systems level critically depends on a detailed knowledge of their expression pattern, their pharmacology and the modes of signal transduction. In this context, compounds acting as agonists, antagonists or positive or negative modulators on TAARs, as well as transgenic animal models overexpressing the TAAR1 receptor are essential tools for dissecting the molecular function of this receptor family and to fully understand their potential relevance as targets in drug development.

The present invention therefore provides genetic constructs comprising a DNA sequence encoding TAAR1 operatively linked to a promoter.

The sequence of TAAR1 gene can be derived from any animal. Preferably the sequence of taar1 derives from a mammal, more preferably, the sequence of taar1 is a human (NM_138327, SEQ. ID NO:1)or a mouse sequence (NM_053205, SEQ. ID NO: 2).

The promoter may be a neuronal promoter. In a preferred embodiment the promoter is a tissue-specific promoter. A tissue-specific promoter may be any promoter, which controls and directs expression of a gene in a tissue-specific manner, e.g., in brain tissue (brain-specific), in muscle tissue, in liver tissue, in kidney tissue, etc. Preferably, the promoter provides specific expression in the central nervous system (CNS). Most preferably, the promoter is the mouse Thy1 promoter (Ingraham, H.A., Lawless, G.M., Evans, G.A. (1986) The mouse Thy-1.2 glycoprotein gene: complete sequence and identification of an unusual promoter. JImmunol. 136, 1482-1489).

The promoter may also be a controllable promoter. A controllable promoter may be any promoter, which controls the expression of a transgene in a regulatable and/or inducible fashion, e.g., by addition of specific inducer or repressor substances. Several inducible bacterial promoter are known in the art (Schultze N, Burki Y, Lang Y, Certa U, Bluethmann H; Nat Biotechnol 1996; 14(4): 499-503; van der Neut R; Targeted gene disruption: applications in neurobiology; J Neurosci Methods 1997; 71(1): 19-27; Liu HS, Lee CH, Lee CF, Su IJ, Chang TY; Lac/Tet dual-inducible system functions in mammalian cell lines. Biotechniques. 1998; 24(4): 624-8, 630-2).

The term "promoter" as used herein refers to DNA regions which control the expression of a coding sequence.

The term "transgenic DNA" as used herein describes DNA artificially introduced and incorporated into an organism.

The term "sequence of *taar1* gene" as used herein, describes the DNA sequence encoding TAAR1.

The genetic construct may additionally comprises a Kozak Sequence. Preferably, the genetic construct comprises an optimized Kozak sequence such as for example the sequence: tccacc. The Kozak Sequence is a DNA sequence that surrounds the ATG start signal for the translation of an mRNA (Kozak, M., An analysis of 5'-noncoding sequences from 699 vertebrate messenger RNAs. Nucleic Acids Res. 15 (1989) 8125-8148.).

In a preferred embodiment, the genetic construct comprise additionally an N-terminal influenza hemaglutinin viral leader sequence. In another preferred embodiment the genetic construct comprises additionally an epitope marker such as for example M1-FLAG epitope. In another preferred embodiment, the genetic construct comprise additionally a Met-Gly linker. In a even more preferred embodiment the genetic construct comprises an N-terminal influenza hemaglutinin viral leader sequence, an M1-FLAG epitope and a Met-Gly linker (Bunzow, et al. (2001) Mol. Pharmacol. 60, 1181-1188; X.M. Guan, T.S. Kobilka, B.K. Kobilka (1992) Enhancement of membrane insertion and function in a type IIIb membrane protein following introduction of a cleavable signal peptide. J. Biol. Chem. 267, 21995-21998).

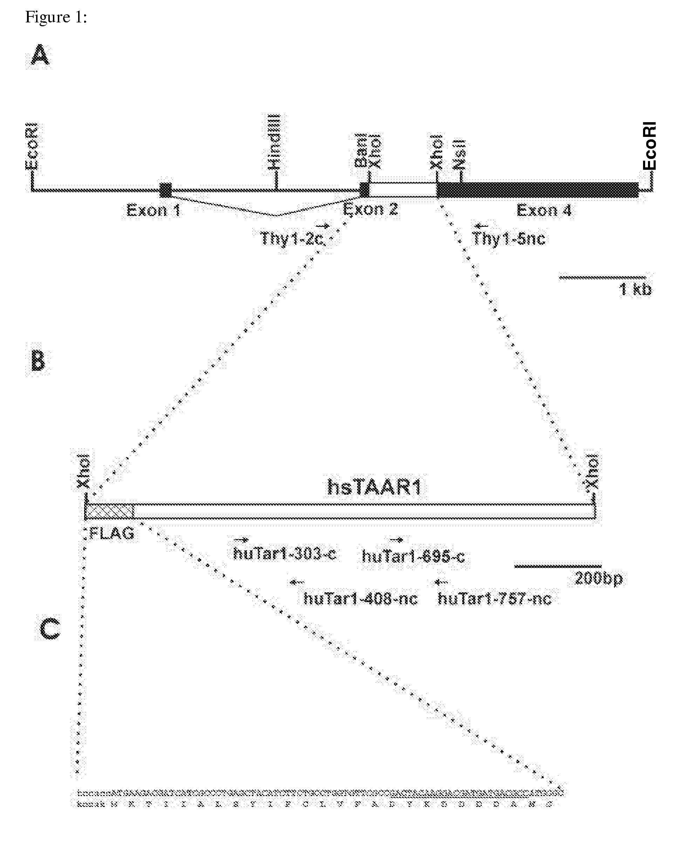

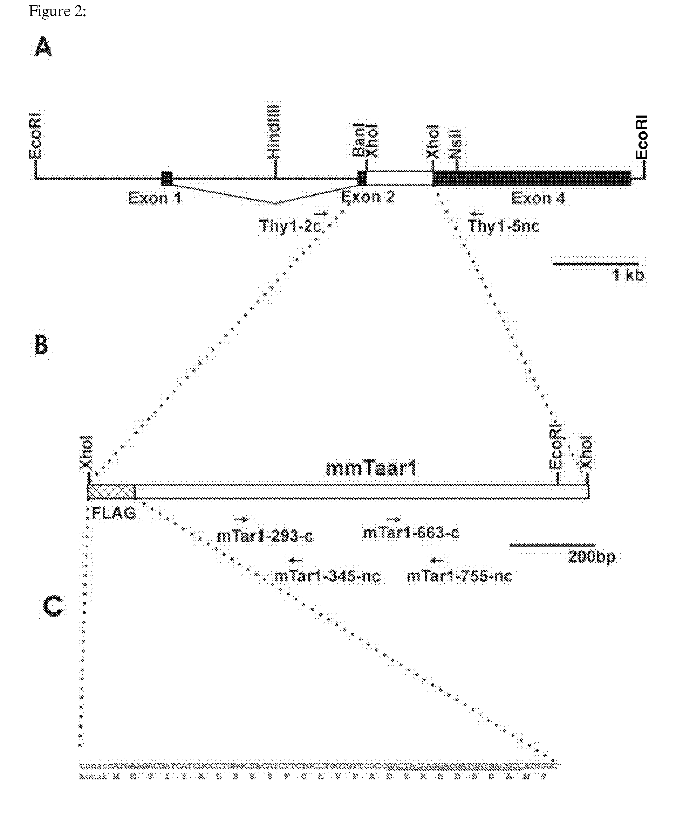

Preferably, the genetic construct is as depicted in Figure 1 or Figure 2.

More preferably, the genetic construct is Thy1-hsTAAR1 incorporated in the plasmid pThy1-hsTAAR1 deposited under accession number DSM 17761 (deposited under the Budapest Treaty at the Deutsche Sammlung von Microorganismen und Zellkulturen GmbH (DSMZ) with an effective deposition date of 30.11.2005), or the genetic construct is Thy1-mmTAAR1 incorporated in the plasmid pThy1-mmTaar1 deposited under accession number DSM 17760 (deposited under the Budapest Treaty at the Deutsche Sammlung von Microorganismen und Zellkulturen GmbH (DSMZ) with an effective deposition date of 30.11.2005).

The present invention further provides a method of producing a transgenic non-human animal whose genome comprise transgenic DNA encoding TAAR1, comprising
a) introducing a genetic construct as described above into a non-human zygote or an non-human embryonic stem cell,
b) generating a transgenic non-human animal from said zygote or embryonic stem cell, and thereby,
c) producing a transgenic non-human animal whose genome comprises transgenic DNA encoding TAAR1.

A further embodiment of the invention provides a method of producing a non-human transgenic animal expressing transgenic TAAR1 comprising
a) introducing a genetic construct as described above into a non-human zygote or an non-human embryonic stem cell,
b) generating a transgenic non-human animal from said zygote or embryonic stem cell, and thereby,
c) producing a transgenic non-human animal expressing transgenic TAAR1.

The zygote or embryonic stem cell may derive from any non-human animal. Preferably, the zygote or embryonic stem cell derives from a rodent. More preferably, the zygote or embryonic stem cell derives from a mouse.

Preferably, the zygote used in the methods described above is a C57BL/6J zygote. Zygotes used in the art which may also be used in the methods of this invention comprise, but are not limited to, CBA/ zygotes, BALB/c zygotes, DBA/2 zygotes and SV129 zygotes.

Embryonic stem cells used in the art which may also be used in the methods of this invention comprise but are not limited to embryonic stem cells derived from mouse strains such as C57BL/6, CBA/, BALB/c, DBA/2 and SV129. Preferably, embryonic stem cells derived from C57BL/6 mice are used (Seong, E et al (2004) Trends Genet. 20, 59-62; Wolfer, D.P. et al., TrendsNeurosci. 25 (2002): 336-340).

The present invention further provides the transgenic non-human animal produced by any of the above described methods.

The term "transgenic TAAR1" as used herein describes TAAR1 protein originating from DNA artificially introduced and incorporated into an organism.

The term "transgenic animal" as used herein describes an animal comprising transgenic DNA in their genome. This transgenic DNA may be incorporated somewhere in the genome.

For example, the transgenic animals may be generated by injecting above described DNA construct into the pronucleus of zygotes, transferring these injected zygotes into pseudo-pregnant foster mothers, breeding founder animals resulting from the oocytes to wild type animals, testing the offspring resulting from these breedings for the presence of the synthetic DNA transgene construct, breeding hemizygous animals, optionally to generate homozygous transgenic animals.

Alternatively, the transgenic animals may be generated by introducing the genetic construct as described above into embryonic stem cells and subsequently selecting embryonic stem cell clones for the presence of the transgene in the genome, verifying the presence of the transgene in the transformed embryonic stem cell clones, injecting the verified recombinant embryonic stem cells into blastocysts of wild type animals, transferring these injected blastocysts into pseudo-pregnant foster mothers, breeding chimeras resulting from the blastocysts to wild type animals, testing the offspring resulting from these breedings for the presence of the transgene, breeding hemizygous animals, optionally to generate homozygous transgenic animals.

In one embodiment of the invention, transgenic non-human animals whose genome comprises transgenic DNA encoding TAAR1 are provided. In a preferred embodiment the transgenic non-human animal comprises a genetic construct as depicted in Fig. 1 or a genetic construct as depicted in Fig. 2.

In another preferred embodiment, the transgenic non-human animal comprises a genetic construct Thy1-hsTAAR1 incorporated in the plasmid pThy1-hsTAAR1 deposited under accession number DSM 17761 (deposited under the Budapest Treaty at the Deutsche Sammlung von Microorganismen und Zellkulturen GmbH (DSMZ) with an effective deposition date of 30.11.2005), or the genetic construct is Thy1-mmTaaR1 incorporated in the plasmid pThy1-mmTaar1 deposited under accession number DSM 17760 (deposited under the Budapest Treaty at the Deutsche Sammlung von Microorganismen und Zellkulturen GmbH (DSMZ) with an effective deposition date of 30.11.2005).

In another embodiment transgenic non-human animals expressing transgenic TAAR1 are provided. In a preferred embodiment, the transgenic TAAR1 is expressed in a tissue-specific manner, i.e., in the brain. In another embodiment, the expression of the transgenic TAAR1 may be temporally controlled, e.g., by addition of specific inducer or repressor substances. In a preferred embodiment, the transgenic non-human animal overexpresses the transgenic TAAR1 protein. In a more preferred embodiment, the transgenic TAAR1 is overexpressed in tissue-specific manner.

The transgenic non-human animal may be any non-human animal known in the art, which maybe used for the methods of the invention. Preferably, the transgenic non-human animal is a mammal, more preferably the transgenic non-human animal is a rodent. Most preferably, the transgenic animal of the invention is a mouse.

The transgenic non-human animals described above may be analyzed genetically, molecularly and behaviorally.

The present invention also relates to descendants of the transgenic non-human animals as provided by the invention, obtained by breeding with the same or with another genotype. A descendant is used herein as a synonym for progeny.

The transgenic animals may be used for preparing primary cell cultures, and for the preparation of secondary cell lines derived from primary cell preparations of these animals or their descendants. Furthermore, the transgenic animals can be used for the preparation of tissue or organotypic brain slice cultures. Moreover, the transgenic animals can be used for the preparation of tissue explants or organ explants or cultures thereof. In addition, the transgenic animal may be used for the preparation of tissue or cell extracts such as membrane or synaptosomal preparations.

The present invention further provides a cell line or primary cell culture derived from the transgenic non-human animals as provided by the invention or their descendants.

Furthermore, the present invention provides a tissue or organotypic brain slice culture, derived from the transgenic non-human animals as provided by the invention or their descendants.

In addition, the present invention provides tissue explants or organ explants or cultures thereof, derived from the transgenic non-human animals as provided by the invention or their descendants.

Moreover, the present invention provides a tissue extract or a cell extract, derived from the transgenic non-human animals as provided by the invention or their descendants.

Cell culture based models can be prepared by two methods. Cell cultures can be isolated from the non-human transgenic animals or prepared from established cell cultures using the same genetic constructs as described above with standard cell transfection techniques.

Integration of the genetic construct comprising transgenic DNA encoding TAAR1, can be detected by various methods comprising genomic Southern blot and PCR analysis using DNA isolated from tissue biopsies of the animals.

It will be apparent to the person skilled in the art that there are a large number of analytical procedures which may be used to detect the expression of the transgenic DNA comprising methods at the RNA level comprising mRNA quantification by reverse transcriptase polymerase chain reaction (RT-PCR) or by Northern blot, *in situ* hybridization, as well as methods at the protein level comprising histochemistry, immunoblot analysis and binding studies. Quantification of the expression levels of the transgene can moreover be determined by the ELISA technology, which is common to those knowledgeable in the art.

Quantitative measurement can be accomplished using many standard assays. For example, transcript levels can be measured using RT-PCR and hybridization methods including RNase protection, Northern blot analysis, and RNA dot blot analysis. Protein levels can be assayed by ELISA, Western blot analysis, and by comparison of immunohistochemically or histochemically stained tissue sections. Immunohistochemical staining, enzymatic histochemical stainings as well as immunoelectron microscopy can also be used to assess the tissue distribution of the endogenous as well as of the overexpressed TAAR1 protein.

The transgenic animals of the invention may be further characterized by methods known in the art, comprising immunohistochemistry, electron microscopy, Magnetic Resonance Imaging (MRI), Positron Emission Tomography (PET) and by behavioral and physiological studies addressing neurological, sensory, and cognitive functions as well as physiological (e.g. metabolic) parameters. Examples of behavioral tests and physiological examinations are: Spontaneous behavior, behavior related to cognitive functions, pharmacologically-disrupted behavior, grip strength test, horizontal wire test, forced swim test, rotarod test, locomotor activity test, prepulse inhibition test, Morris water maze test, Y-maze test, light-dark preference test, passive and active avoidance tests, marble burying test, plus maze test, learned helplessness test, stress-induced hyperthermia, measuring food consumption and development of body weight over time, measuring body temperature and energy consumption under resting and basal conditions and during heat and cold exposure, determining the thermoneutral zone, determining the food assimilation coefficient (e.g. by bomb calorimetry), determining the energy assimilation and the energy content of feces, determining the respiratory coefficient e.g. for analysis of the carbohydrate and lipid metabolism, determining the substrate utilization and energy expenditure during food restriction, determining the oxygen consumption, CO₂- and heat production e.g. by indirect calorimetry, measuring the heart rate and blood pressure under resting, basal and stress conditions (e.g. by telemetry), determining the body composition (e.g. regarding water content, fat amount and fat-free mass).

A further objective of the present invention is the use of a non-human transgenic animal, a cell line or primary cell culture, a tissue or organotypic brain slice cultures, a tissue explant or an organ explant or culture thereof, a tissue or cell extract derived from the transgenic non-human animals as provided by the invention or its descendants as a model for identifying and testing the therapeutic effect of a compound in disorders comprising depression, anxiety disorders, bipolar disorder, attention deficit hyperactivity disorder, stress-related disorders such as posttraumatic stress disorder, psychotic disorders such as schizophrenia, neurological diseases such as Parkinson's Disease, neurodegenerative disorders such as Alzheimer's Disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

Moreover, the present invention provides the use of a non-human transgenic animal, a cell line or primary cell culture, a tissue or organotypic brain slice cultures, a tissue explant or an organ explant or culture thereof, a tissue or cell extract derived from the transgenic non-human animals as provided by the invention or its descendants as a model for studying the susceptibility to compounds inducing disorders comprising depression, anxiety disorders, bipolar disorder, attention deficit hyperactivity disorder, stress-related disorders such as posttraumatic stress disorder, psychotic disorders such as schizophrenia, neurological diseases such as Parkinson's Disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

A further objective of the present invention is the use of a non-human transgenic animal, a cell line or primary cell culture, a tissue or organotypic brain slice cultures, a tissue explant or an organ explant or culture thereof, a tissue or cell extract derived from the transgenic non-human animals as provided by the invention or its descendants as a tool for assessing TAAR1 function.

Furthermore, the present invention provides the use of a non-human transgenic animal, a cell line or primary cell culture, a tissue or organotypic brain slice cultures, a tissue explant or an organ explant or culture thereof, a tissue or cell extract derived from the transgenic non-human animals as provided by the invention or its descendants as a tool for identifying unknown ligands of TAAR1 and their characterization.

Moreover, the present invention provides the use of a non-human transgenic animal, a cell line or primary cell culture, a tissue or organotypic brain slice cultures, a tissue explant or an organ explant or culture thereof, a tissue or cell extract derived from the transgenic non-human animals as provided by the invention or its descendants as a tool for determining the specificity of compounds acting on TAAR1.

A further objective of the present invention is the use of a non-human transgenic animal, a cell line or primary cell culture, a tissue or organotypic brain slice cultures, a tissue explant or an organ explant or culture thereof, a tissue or cell extract derived from the transgenic non-human animals as provided by the invention or its descendants for studying the intracellular trafficking of TAARs or of other cellular components linked to TAARs.

The invention further provides genetic constructs, methods, transgenic animals, test systems and uses substantially as described herein before especially with reference to the foregoing examples.

Having now generally described this invention, the same will become better understood by reference to the specific examples, which are included herein for purpose of illustration only and are not intended to be limiting unless otherwise specified, in connection with the following figures.

### Figures:

Figure 1 shows a schematic representation of the genetic construct huTAAR-TG incorporated in the plasmid pThy1-hsTAAR1 (not shown). The control region of the Thy1 gene was used to control the expression of a human TAAR1 cDNA in the brain.
A: The 6.8 kb control region of the Thy1 gene in which a 1.5 kbp BanI-XhoI fragment located on exons 2 and 4 was replaced by a XhoI cloning site thereby deleting Exon 3. The present exons (Exon 1, Exon 2, Exon 4) of the Thy1 gene are indicated in black.
B: The XhoI site in the Thy1 gene was used to insert an XhoI fragment containing the human TAAR1 cDNA (hsTAAR1, white). The human TAAR1 cDNA was fused with a N-terminal tag (FLAG, hatched).
C: The N-terminal tag is shown as nucleotide sequence and as its translation into single letter amino acid code. The Kozak sequence (small letters) is followed by a signal sequence of the influenza hemaglutinin virus (capital letters), the FLAG epitope tag (capital letters, underlined) and the Met-Gly linker (capital letters, italic).

Figure 2: shows a schematic representation of the mmTAAR-TG construct incorporated in the plasmid pThy1-mmTaar1 (not shown). The control region of the Thy1 gene was used to control the expression of a human Taar1 cDNA in the brain.
A: The 6.8 kb control region of the Thy1 gene in which a 1.5 kbp BanI-XhoI fragment located on exons 2 and 4 was replaced by a XhoI cloning site thereby deleting Exon 3. The present exons (Exon 1, Exon 2, Exon 4) of the Thy1 gene are indicated in black.
B: The XhoI site in the Thy1 gene was used to insert an XhoI fragment containing the human Taar1 cDNA (hsTaar1, white). The human Taar1 cDNA was fused with a N-terminal tag (FLAG, hatched).
C: The N-terminal tag is shown as nucleotide sequence and as its translation into single letter amino acid code. The Kozak sequence (small letters) is followed by a signal sequence of the influenza hemaglutinin virus (capital letters), the FLAG epitope tag (capital letters, underlined) and the Met-Gly linker (capital letters, italic).

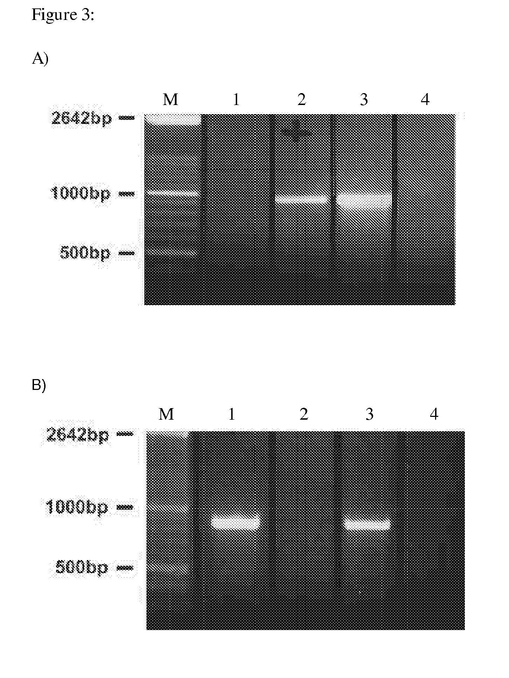

Figure 3: shows the genotyping of transgenic lines and B6-Tg(TAAR1) 1 and B6-Tg(Taar1)2 by means of PCR. DNA was analyzed for the presence of the transgene, indicating the respective genotypes of the animals. Marker XIV (Roche) was used as molecular weight standard.
A: B6-Tg(Taar1)2: Ethidium bromide stained agarose gel of PCR reaction to detect the presence of the Thy1-mmTaar1 sequences in tail biopsies of mice.
M: Marker XIV, 1: mouse biopsy 9; 2: mouse biopsy 10; 3: positive control; 4: negative control.
B: B6-Tg(TAAR1)1: Ethidium bromide stained agarose gel of PCR reaction to detect the presence of the Thy1-hsTaar1 sequences in tail biopsies of mice.
M: Marker XIV, 1: mouse biopsy 34; 2: mouse biopsy 35; 3: positive control; 4: negative control.

### Examples

Commercially available reagents referred to in the examples were used according to manufacturer's instructions unless otherwise indicated.

### Example 1: Construction of the pThy1-hsTAAR1 and pThy1-mmTaar1 plasmid constructs

### PCR amplification of the cDNA sequences

Oligonucleotides were designed based on the published mouse TAAR1 coding sequence (Genbank Accession No. NM_053205, SEQ. ID NO: 2) or human TAAR1 coding sequence (Genbank Accession No. NM_138327, SEQ. ID NO: 1) or on other elements of the p Thy1-hsTAAR1 and pThy1-mmTaar1 plasmids and they were obtained from Microsynth AG (Balgach, Switzerland). All molecular cloning techniques were carried out essentially according to Sambrook et. al. (Molecular Cloning: A laboratory manual. 1989, Cold Spring Harbor Laboratory Press, Cold Spring Harbor.) and the instructions of the suppliers of kits and enzymes.

The coding sequences of human (hsTAAR1) and mouse (mmTaar1) were amplified by PCR from genomic C57BL/6 DNA (mmTaar1) or a cloned coding sequence pcDNA3.1-humTar01 (hsTAAR1).

The 5'-oligonucleotides, hTar1-tg-3c (SEQ. ID NO: 21) and mTar1-tg-1c (SEQ. ID NO: 27), were designed to contain a XhoI site, a Kozak sequence, sequences coding for a signal sequence of the influenza hemaglutinin virus, the FLAG epitope Tag and a Met-Gly linker (Fig. 1C, Fig. 2C). The 3'-oligonucleotides, hTar1-tg-4nc (SEQ. ID NO: 22) and mTar1-tg-2nc (SEQ. ID NO: 28) introduce a XhoI site immediately downstream of the stop codon.

### • hsTAAR1-PCR amplification

*0.5 ng*/*µl template DNA (pcDNA3.1-humTar01), 300 µM dNTPs (PCR Nucleotide Mix, Roche Diagnostics, Mannheim, Germany), 1 µM of oligonucleotide huTar1-tg-3c, 1µM of oligonucleotide huTarl -tg-4nc 1x PCR buffer 3 (part of the Expand High Fidelity PCR System; Roche Diagnostics, Mannheim, Germany), 0,075 U*/*µl Expand Polymerase Mix (part of the Expand High Fidelity PCR System; Roche Diagnostics, Mannheim, Germany)) in a total volume of 20 µl were incubated with the following protocol: 94°C 2 min., 10× (94°C 10 sec., 60°C 30 sec., 68°C 2 min.), 20× (94°C 10 sec., 60°C 30 sec., 68°C 2 min.+15 sec*/*cycle) 68°C 7 min, ∞ 12°C on a PCR Thermocycler M J Research PTC-200 (MJResearch Inc., Watertown, USA).*

### • mmTaar1-PCR amplification

*0.5 ng*/*µl template DNA (genomic C57BL*/*6 DNA), 300µM dNTPs (PCR Nucleotide Mix, Roche Diagnostics, Mannheim, Germany), 1 µM of oligonucleotide mTar1-tg-1c, 1µM of oligonucleotide mTar1-tg-2nc, 1x PCR buffer 3 (part of the Expand High Fidelity PCR System; Roche Diagnostics, Mannheim, Germany), 0,075 U*/*µl Expand Polymerase Mix (part of the Expand High Fidelity PCR System; Roche Diagnostics, Mannheim, Germany)) in a total volume of 20µl were incubated with the following protocol: 94°C 2 min., 10× (94°C 10 sec., 60°C 30 sec., 68°C 2 min.), 20× (94°C 10 sec., 60°C 30 sec., 68°C 2 min.+15 sec*/*cycle) 68°C 7 min, ∞ 12°C on a PCR Thermocycler MJ Research PTC-200 (MJResearch Inc., Watertown, USA).*

### Cloning into the cloning vector pCMV

The resulting 1.1kb PCR amplification products were separated using agarose gel-electrophoresis followed by purification with the StrataPrep PCR Purification Kit (Stratagene, La Jolla, CA, USA). The purified PCR products were then subcloned into the SrfI site of the pCMVScript vector of the pCMV PCR Cloning Kit (Invitrogen-Gribco, Carlsbad, CA, USA) according to the kits instructions. The resulting vector are pCMV-mmTaar1 and pCMV-hsTAAR1.

Orientation and sequence were confirmed by sequencing using the BigDye Terminator v1.1 Cycle Sequencing Kit (Applied Biosystems) and an ABIPrism 310 Genetic Analyzer with the oligonucleotides depicted in table 1:

**Table 1: Sequencing reactions of pCMV-mmTaar1 and pCMV-hsTAAR1**

| Vector | Oligonucleotides used for sequencing | | Resulting sequence: |
|---|---|---|---|
| | Term | SEQ. ID NO: | |
| pCMV-hsTAAR1 | T3 forward | 29 | SEQ. ID NO: 5 |
| pCMV-hsTAAR1 | T7 reverse | 30 | SEQ. ID NO: 6 |
| pCMV-hsTAAR1 | hu-Tar 303c | 17 | SEQ. ID NO: 7 |
| pCMV-hsTAAR1 | hu-Tar 408nc | 18 | SEQ. ID NO: 8 |
| pCMV-hsTAAR1 | hu-Tar 695 c | 19 | SEQ. ID NO: 9 |
| pCMV-hsTAAR1 | hu-Tar 757 nc | 20 | SEQ. ID NO: 10 |
| pCMV-mmTaar1 | T3 forward | 29 | SEQ. ID NO: 11 |
| pCMV-mmTaar1 | T7 reverse | 30 | SEQ. ID NO: 12 |
| pCMV-mmTaar1 | mTar-293c | 23 | SEQ. ID NO: 13 |
| pCMV-mmTaar1 | mTar-345nc | 24 | SEQ. ID NO: 14 |
| pCMV-mmTaar1 | mTar-663c | 25 | SEQ. ID NO: 15 |
| pCMV-mmTaar1 | mTar-755nc | 26 | SEQ. ID NO: 16 |

The sequences of SEQ. ID. NOs: 5-10 were edited manually and the vector sequence of pCMV was omitted. They can be assembled to confirm the correct sequence of the pCMV-hsTAAR1 vector (SEQ. ID NO: 3).

The sequences of SEQ. ID. NOs: 11-16 were edited manually and the vector sequence of pCMV was omitted. They can be assembled to confirm the correct sequence of the pCMV-mmTaar1 vector (SEQ. ID NO: 4).

### Cloning into the pThy vector

The vector pCMV-mmTaar1 and pCMV-hsTAAR1 were used to generate the vectors pThy1-mmTaar1 and pThy1-hsTAAR1, respectively. To this end a pCMV-mmTaar1 and pCMV-hsTAAR1 were digested with XhoI and the resulting 1.1 kb fragment containing the Taar1 coding sequences were purified from agarose gels. They were subsequently ligated into the vector pThy1 (Fig. 1A; Fig. 2A; Richards, J.G., Higgins, G.A., Ouagazzal, A.M., Ozmen, L., Kew, J.N., Bohrmann, B., Malherbe, P., Brockhaus, M., Loetscher, H., Czech, C., Huber, G., Bluethmann, H., Jacobsen, H. and Kemp, J.A. (2003) J. Neurosci. 23(26), 8989-9003)) that has been opened with the restriction enzyme XhoI.

The resulting vectors pThy1-mmTaar1 and pThy1-hsTAAR1 were confirmed using sequencing with Thy1-2c (SEQ. ID NO: 31) and restriction digests.

Deposition data: The plasmid comprising the genetic construct pThy1-mmTaar1 was deposited under the Budapest Treaty at the Deutsche Sammlung von Microorganismen und Zellkulturen GmbH (DSMZ), Mascheroder Weg 1 b, D-38124 Braunschweig, Germany, with an effective deposition date of 30.11.2005 under the accession number DSM 17760.

The plasmid comprising the genetic construct pThy1-hsTAAR1 was deposited under the Budapest Treaty at the Deutsche Sammlung von Microorganismen und Zellkulturen GmbH (DSMZ), Mascheroder Weg 1 b, D-38124 Braunschweig, Germany, with an effective deposition date of 30.11.2005 under the accession number DSM 17761.

### Example 2: Generating transgenic mouse lines B6-Tg(TAAR1) 1 and B6-Tg(Taar1)2

All handling of animals was carried out in compliance with Swiss Federal and Cantonal laws on animal research, and permission for the generation and handling of the mutant mouse line was specifically granted from the Kantonale Veterinäramt of Basel City with the Tierversuchgenehmigung No. 192.

The plasmids pThy1-mmTaar1 and pThy1-hsTAAR1 were digested with NotI and PvuI, followed by gel purification of the 7,6kb fragment containing the Thy1 promotor and Taar1 sequences. These fragments of pThy1-mmTaar1 and pThy1-hsTAAR1 were injected into the pronucleus of fertilized C57BL/6 oocytes which were subsequently transferred into the oviducts of pseudopregnant foster mothers essentially as described in (Gene Targeting. 1999, Second Edition, The Practical Approach Series, Oxford University Press, New York) and Hogan, B., Beddington, R., Costantini, F., und Lacy, E. (Manipulating the mouse embryo. 1994, Second Edition, Cold Spring Harbor Press, Cold Spring Harbor). Offspring delivered by the foster mothers were considered as founder animals and analyzed for the presence of the respective transgenes in the genome.

To this end, genomic DNA was isolated from tail biopsies of adult animals with a MagNAPure LC system for nucleic acid purification (Roche Applied Science, Rotkreuz, Switzerland) according to the instructions of the manufacturer and analyzed for the presence of the TAAR1 transgenes by means of PCR.

Transgenic animals comprising in their genome the genetic construct "Thy1-hsTAAR1" are designated as B6-Tg(TAAR1)1 and transgenic animals comprising in their genome the genetic construct "Thy1-mmTaar1" are designated as B6-Tg(Taar1)2.

### • Genotyping of B6-Tg(TAAR1)1

*0.5 ng*/*µl tail biopsy DNA, 200 µM dNTPs (PCR Nucleotide Mix, Roche Diagnostics, Mannheim, Germany), 1 µM of oligonucleotide Thy1-2c(SEQ. ID. NO: 31), 1 µM of oligonucleotide huTar1-757-n (SEQ. ID NO:20)c, 1x PCR reaction buffer (Roche Diagnostics, Mannheim, Germany), 0,05 U*/*µl Taq DNA Polymerase (Roche Diagnostics, Mannheim, Germany)) in a total volume of 20 µl were incubated with the following protocol: 94°C 2 min., 30x (94°C 20 sec., 60°C 30 sec., 72°C 50 sec) 72°C 7 min, ∞ 12°C on a PCR Thermocycler MJResearch PTC-200 (MJResearch Inc., Watertown, USA).*

### • GenotypingofB6-Tg(Taarl)2

*0.5 ng*/*µl tail biopsy DNA, 200 µM dNTPs (PCR Nucleotide Mix, Roche Diagnostics, Mannheim, Germany), 1 µM of oligonucleotide mTarl-293-c, 1 µM of oligonucleotide Thy1-5-nc (SEQ. ID NO: 32), 1x PCR reaction buffer (Roche Diagnostics, Mannheim, Germany), 0,05 U*/*µl Taq DNA Polymerase (Roche Diagnostics, Mannheim, Germany)) in a total volume of 20 µl were incubated with the following protocol: 94°C 2 min., 30x (94°C 20 sec., 64°C 30 sec., 72°C 50 sec) 72°C 7 min, ∞ 12°C on a PCR Thermocycler MJResearch PTC-200 (MJ Research Inc., Watertown, USA).*

The PCR products were analyzed by standard agarose gel electrophoresis as described in Sambrook et al. (1989). See figure 3 for an example of genotyping.

Three founders were identified with an insertion of the human TAAR1 construct and designated as B6-Tg(TAAR1) 1.6, B6-Tg(TAAR1) 1.7 and B6-Tg(TAAR1) 1.29.

Three founders were identified with an insertion of the murine Taar1 construct and designated as B6-Tg(Taar1)2.15, B6-Tg(Taar1)2.19 and B6-Tg(Taar1)2.27.

Semiquantitative analysis of human TAAR1 and mouse TAAR1 expression, respectively, in brain regions of the transgenic mouse lines B6-Tg(TAAR1)1 and B6-Tg(Taar1)2

Offspring of the identified founders B6-Tg (TAAR1) 1.6, B6-Tg(TAAR1) 1.7 and B6-Tg(TAAR1) 1.29 were tested for expression of mRNA coded by the constructs Thy1-hsTAAR1. Offspring of the identified founders B6-Tg(Taar1)2.15, B6-Tg(Taar1)2.19 and B6-Tg(Taar1)2.27 were tested for expression of mRNA coded by the constructs Thy1-mm(Taar1).

To test for expression of the constructs Thy1-mm(Taar1) and Thy1-hsTAAR1 a real time PCR assay was used. To this end total RNA of mouse brains was isolated, transcribed into cDNA and detected with Real-Time PCR using a combination of primers and probe specific for the synthetic N-terminal Tag common to both constructs (Fig 1C, Fig 2C).

RNA was prepared from tissue samples essentially according to Chomczynski and Sacchi (Single-step method of RNA isolation by acid guanidinium thiocyanate-phenolchloroform extraction, Anal. Biochem. 162 (1987) 156-159.). In brief, mice 6 to 8 weeks of age were sacrificed, and total brains were removed and homogenized in 10× volumes of Trizol Reagent (Invitrogen, Paisley, UK) in Lysing Matrix D tubes using a Fast Prep FP120 tissue homogenizer (Savant Instruments, Inc., Holbrook, N.Y.) at a setting of 6 for 20 s. The homogenates were extracted with 1/5 volume of Chloroform and subsequently precipitated using 2 volumes of isopropanol. The precipitated total RNA was washed with 80% Ethanol and dissolved in DEPC-H₂O. 100µg Total RNA was further purified using the RNeasy Mini Protocol for RNA Cleanup of the RNeasy Mini Kit (Qiagen, Hilden, Germany) including a on-column DNase Digestion with the RNase-Free DNase Set (Qiagen, Hilden, Germany).

For cDNA synthesis the Omniscript Reverse Transkription Kit (Qiagen, Hilden, Germany) was used according to the instructions of the manufacturer. Briefly, 2µg total RNA were mixed with 1µM oligo (dT) 15 (Roche Diagnostics, Mannheim, Germany), 0.5mM of each dNTP (PCR Nucleotide Mix, Roche Diagnostics, Mannheim, Germany), 1x Buffer RT (Qiagen, Hilden, Germany), 10 U RNAse Inhibitor (Roche Diagnostics, Mannheim, Germany) 1 µl Omniscript Reverse Transcriptase (Qiagen, Hilden, Germany) in a total volume of 20 µl. The reaction mix was incubated at 37°C for 1h.

Real time PCR was carried out using Custom designed Quantitect Gene expression assays according to the Quantitect Gene Expression Assay Handbook (Qiagen, Hilden, Germany). The transgene specific Quantitect Custom assay was designed to detect the synthetic N-terminal tag and consisted of a forward primer (SEQ. ID NO: 33), reverse Primer (SEQ. ID NO: 34) and FAM labeled probe (SEQ. ID NO: 35). As control the Yakima Yellow labeled QuantiTect Endogenous Control Assay (Mm Arbp 1 YY Quantitect Gene Expression Assay, Qiagen, Hilden, Germany) against the housekeeping gene murine Arbp (Acidic ribosomal phospoprotein PO) was used. 10 µl QuantiTect Probe PCR Master Mix, 2µl Quantitect Assay Mix and 2µl cDNA were set up in white reaction plates (MJ Research Inc., Watertown, USA) on a DNA Engine Opticon 2 (MJ Research Inc., Watertown, USA) and real time PCR with the following temperature protocol were performed: 95°C 15 min., 45x (94°C 15 sec., 56°C 30 sec., 76°C 30 sec). C(T) values were calculated using the Opticon monitor Software (MJ Research Inc., Watertown, USA).

The results of one experiment are summarized in Table 2. In this experiment two mice each of the lines B6-Tg (TAAR1) 1.6, B6-Tg(TAAR1) 1.7, B6-Tg(TAAR1) 1.29, B6-Tg(Taar1)2.15, B6-Tg(Taar1)2.19 and B6-Tg(Taar1)2.27 as well as two wildtype mice were sacrificed, cDNA was produced as described above and two PCR reactions were run for each mouse with the transgene specific Quantitect assay (Qiagen, Hilden, Germany) and, as control, one PCR reaction was run for each mouse with the Arbp specific Quantitect assay (see Table 2).

From wildtype mice, control reactions where the reverse transcriptase was not added, as well as water controls, C(T) values are obtained close or below to the threshold of detection (Table 2). On the other hand, the lines B6-Tg(TAAR1) 1.7, B6-Tg(TAAR1)1.29, B6-Tg(Taar1)2.19 and B6-Tg(Taar1)2.27 express the transgenic construct, as C(T) values are within the range of plasmid positive controls (Table 2).

**Table 2: Results of the real time PCR analysis of cDNAs derived from B6-Tg(TAAR1) 1 and B6-Tg(Taar1)2 mouse brains for the presence of the mRNA transcripts. (C(T): number of PCR cycles; nd: not determined).**

| Sample: | Transgene specific probe: C(T) +/- standard deviation | Housekeeping gene (Arbp) probe: C(T) +/- standard deviation |
|---|---|---|
| B6-Tg (TAAR1)1.6 | 36.4 +/- 4.2 | 30.7 +/- 0.6 |
| B6-Tg(TAAR1)1.7 | 24.6 +/- 0.8 | 28.5 +/- 2.4 |
| B6-Tg(TAAR1)1.29 | 27.2 +/- 1.6 | 31.6 +/- 2.2 |
| B6-Tg(Taar1)2.15 | 36.2 +/- 3.4 | 29.6 +/- 0.1 |
| B6-Tg(Taar1)2.19 | 23.7 +/- 0.4 | 30.0 +/- 1.8 |
| B6-Tg(Taar1)2.27 | 24.9 +/- 0.6 | 28.0 +/- 1.3 |
| wildtype | 39.6 +/- 3.5 | 28.1 +/- 1.1 |
| Blank (H₂O) | below threshold | nd |
| B6-Tg(Taar1)2.19 without reverse transcription | below threshold | nd |
| B6-Tg(TAAR1)1.7 without reverse transcription | below threshold | nd |
| pThy1-mmTaar1 (10³ copies) | 33.6 | nd |
| pThy1-mmTaar1 (10⁴ copies) | 29.1 | nd |
| pThy1-mmTaar1 (10⁵ copies) | 25.4 | nd |
| pThy1-mmTaar1 (10⁶ copies) | 22.2 | nd |
| pThy1-mmTaar1 (10⁷ copies) | 19.1 | nd |

## Claims

1. A genetic construct comprising a DNA sequence encoding TAAR1 operatively linked to a promoter.

2. A genetic construct according to claim 1, wherein the coding sequence is a human sequence.

3. A genetic construct according to any one of the claims 1 or 2, wherein the promoter is a tissue-specific promoter.

4. A genetic construct according to any one of the claims 1 to 3 wherein the promoter is a brain-specific promoter.

5. A genetic construct according to any one of the claims 1 to 3, wherein the promoter is a controllable promoter.

6. A method of producing a transgenic non-human animal whose genome comprise transgenic DNA encoding TAAR1, comprising
a) introducing a genetic construct according to any one of the claims 1 to 5 into a non-human zygote or an non-human embryonic stem cell,
b) generating a transgenic non-human animal from said zygote or embryonic stem cell, and thereby,
c) producing a transgenic non-human animal whose genome comprises transgenic DNA encoding TAAR1.

7. A method of producing a non-human transgenic animal expressing transgenic TAAR1 comprising
a) introducing a genetic construct according to any one of the claims 1 to 5 into a non-human zygote or an non-human embryonic stem cell,
b) generating a transgenic non-human animal from said zygote or embryonic stem cell, and thereby,
c) producing a transgenic non-human animal expressing transgenic TAAR1.

8. A transgenic non-human animal produced by the method according to any one of the claims 6 to 7.

9. A transgenic non-human animal, whose genome comprises a genetic construct according to any of the claims 1 to 5.

10. A transgenic non-human animal according to claim 8 or 9 wherein the transgenic animal is a rodent.

11. A transgenic non-human animal according to claim 8 or 9 wherein the transgenic animal is a mouse.

12. A transgenic non-human animal according to claim 8 or 9 wherein the transgenic animal is C57BL/6.

13. A transgenic non-human animal according to any one of the claims 8 to 12 overexpressing TAAR1 protein.

14. A transgenic non-human animal according to any one of the claims 8 to 12 overexpressing TAAR1 protein in a tissue-specific manner.

15. Descendants of the transgenic non-human animal according to any of the claims 8 to 14.

16. A cell line or primary cell culture derived from a transgenic non-human animal or its descendants according to any one of claims 8 to 15.

17. A tissue or organotypic brain slice cultures derived from a transgenic non-human animal or its descendants according to any one of the claims 8 to 15.

18. A tissue explant or a organ explant or a culture thereof, derived from a transgenic non-human animal or its descendants according to any one of the claims 8 to 15.

19. A tissue extract or a cell extract, derived from a transgenic non-human animal or its descendants according to any one of the claims 8 to 15.

20. Use of a non-human transgenic animal according to claims 8 to 15, or a cell line or primary cell culture according to claim 16, or a tissue or organotypic brain slice cultures according to claim 17 or a tissue or an organ explant or culture thereof according to claim 18, or a tissue or cell extract according claim 19 as a model for identifying and testing the therapeutic effect of a compound in disorders comprising depression, anxiety disorders, bipolar disorder, attention deficit hyperactivity disorder, stress-related disorders, psychotic disorders such as schizophrenia, neurological diseases such as Parkinson's Disease, neurodegenerative disorders such as Alzheimer's Disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

21. Use of a non-human transgenic animal according to claims 8 to 15, or a cell line or primary cell culture according to claim 16, or a tissue or organotypic brain slice cultures according to claim 17, or a tissue or an organ explant or culture thereof according to claim 18, or a tissue or cell extract according claim 19 as a tool for assessing TAAR1 function.

22. Use of a non-human transgenic animal according to claims 8 to 15, or a cell line or primary cell culture according to claim 16, or a tissue or organotypic brain slice cultures according to claim 17, or a tissue or an organ explant or culture thereof according to claim 18, or a tissue or cell extract according claim 19 as a tool for identifying unknown ligands of TAAR1.

23. Use of a non-human transgenic animal according to claims 8 to 15, or a cell line or primary cell culture according to claim 16, or a tissue or organotypic brain slice cultures according to claim 17 or a tissue or an organ explant or culture thereof according to claim 18, or a tissue or cell extract according claim 19 as a tool for determining the specificity of compounds acting on TAAR1.

24. Use of a non-human transgenic animal according to claims 8 to 15, or a cell line or primary cell culture according to claim 16, or a tissue or organotypic brain slice cultures according to claim 17, or a tissue or an organ explant or culture thereof according to claim 18, or a tissue or cell extract according claim 19 for studying the intracellular trafficking of TAARs or of other cellular components linked to TAARs.

25. The genetic constructs methods, transgenic animals, test systems and uses substantially as described herein before especially with reference to the foregoing examples.


SEQUENCE LISTING

<110> F. Hoffmann-La Roche

<120> TAAR1 transgenic animal

<130> 23400 EP

<150> EP 05111699.4
<151> 2005-12-05

<160> 35

<170> PatentIn version 3.3

<210> 1
<211> 1020
<212> DNA
<213> Homo sapiens

<400> 1
atgatgccct tttgccacaa tataattaat atttcctgtg tgaaaaacaa ctggtcaaat 60

gatgtccgtg cttccctgta cagtttaatg gtgctcataa ttctgaccac actcgttggc 120

aatctgatag ttattgtttc tatatcacac ttcaaacaac ttcatacccc aacaaattgg 180

ctcattcatt ccatggccac tgtggacttt cttctggggt gtctggtcat gccttacagt 240

atggtgagat ctgctgagca ctgttggtat tttggagaag tcttctgtaa aattcacaca 300

agcaccgaca ttatgctgag ctcagcctcc attttccatt tgtctttcat ctccattgac 360

cgctactatg ctgtgtgtga tccactgaga tataaagcca agatgaatat cttggttatt 420

tgtgtgatga tcttcattag ttggagtgtc cctgctgttt ttgcatttgg aatgatcttt 480

ctggagctaa acttcaaagg cgctgaagag atatattaca aacatgttca ctgcagagga 540

ggttgctctg tcttctttag caaaatatct ggggtactga cctttatgac ttctttttat 600

atacctggat ctattatgtt atgtgtctat tacagaatat atcttatcgc taaagaacag 660

gcaagattaa ttagtgatgc caatcagaag ctccaaattg gattggaaat gaaaaatgga 720

atttcacaaa gcaaagaaag gaaagctgtg aagacattgg ggattgtgat gggagttttc 780

ctaatatgct ggtgcccttt ctttatctgt acagtcatgg acccttttct tcactacatt 840

attccaccta ctttgaatga tgtattgatt tggtttggct acttgaactc tacatttaat 900

ccaatggttt atgcattttt ctatccttgg tttagaaaag cactgaagat gatgctgttt 960

ggtaaaattt tccaaaaaga ttcatccagg tgtaaattat ttttggaatt gagttcatag 1020

<210> 2
<211> 999
<212> DNA
<213> Mus musculus

<400> 2
atgcatcttt gccacgctat cacaaacatt tcccacagaa acagcgactg gtcaagagaa 60

gtccaagctt ccctgtacag cttaatgtca ctcataatcc tggccactct ggttggcaac 120

ttaatagtaa taatttccat atcccatttc aagcaacttc atacacccac caactggctc 180

cttcactcca tggccattgt cgactttctg ctgggctgtc tgataatgcc ctgcagcatg 240

gtgagaactg ttgagcgctg ttggtatttt ggggaaatcc tctgtaaagt tcacaccagc 300

accgatatca tgctgagctc cgcctccatt ttccacttag ctttcatttc cattgaccgc 360

tactgtgctg tgtgtgaccc tttgagatac aaagccaaga tcaatatctc cactattctt 420

gtgatgatcc tcgttagttg gagccttcct gctgtttatg catttgggat gatcttcctg 480

gaactgaact taaaaggagt ggaagagctg tatcgcagtc aggtcagcga cctgggcggc 540

tgttctccct tctttagtaa agtatctggg gtactggcgt tcatgacttc cttctatata 600

cctggatctg ttatgttatt tgtttactat aggatatatt tcatagctaa aggacaagca 660

aggtcaatca atcgtacgaa tgttcaagtt ggattggaag ggaaaagcca agcaccacaa 720

agcaaggaaa caaaagccgc gaagacctta gggatcatgg tgggcgtttt cctcgtatgc 780

tggtgcccgt tctttctctg cacggtcctg gaccctttcc tgggctatgt tatcccaccc 840

tctctgaatg acgcactgta ttggtttggg tacttgaatt ctgccctcaa tccgatggtt 900

tatgcctttt tctatccctg gttcagaaga gccttgaaga tggttctcct tggtaaaatt 960

ttccaaaaag attcatctag gtctaagcta tttttgtaa 999

<210> 3
<211> 1116
<212> DNA
<213> Artificial

<220>
<223> Description of artificial sequence: XhoI fragement of genetic
construct Thy1-hsTAAR1 comprising hsTAAR1 cDNA fused with a
N-terminal FLAG tag.

<400> 3
ctcgagtcca ccatgaagac gatcatcgcc ctgagctaca tcttctgcct ggtgttcgcc 60

gactacaagg acgatgatga cgccatgggc atgatgccct tttgccacaa tataattaat 120

atttcctgtg tgaaaaacaa ctggtcaaat gatgtccgtg cttccctgta cagtttaatg 180

gtgctcataa ttctgaccac actcgttggc aatctgatag ttattgtttc tatatcacac 240

ttcaaacaac ttcatacccc aacaaattgg ctcattcatt ccatggccac tgtggacttt 300

cttctggggt gtctggtcat gccttacagt atggtgagat ctgctgagca ctgttggtat 360

tttggagaag tcttctgtaa aattcacaca agcaccgaca ttatgctgag ctcagcctcc 420

attttccatt tgtctttcat ctccattgac cgctactatg ctgtgtgtga tccactgaga 480

tataaagcca agatgaatat cttggttatt tgtgtgatga tcttcattag ttggagtgtc 540

cctgctgttt ttgcatttgg aatgatcttt ctggagctaa acttcaaagg cgctgaagag 600

atatattaca aacatgttca ctgcagagga ggttgctctg tcttctttag caaaatatct 660

ggggtactga cctttatgac ttctttttat atacctggat ctattatgtt atgtgtctat 720

tacagaatat atcttatcgc taaagaacag gcaagattaa ttagtgatgc caatcagaag 780

ctccaaattg gattggaaat gaaaaatgga atttcacaaa gcaaagaaag gaaagctgtg 840

aagacattgg ggattgtgat gggagttttc ctaatatgct ggtgcccttt ctttatctgt 900

acagtcatgg acccttttct tcactacatt attccaccta ctttgaatga tgtattgatt 960

tggtttggct acttgaactc tacatttaat ccaatggttt atgcattttt ctatccttgg 1020

tttagaaaag cactgaagat gatgctgttt ggtaaaattt tccaaaaaga ttcatccagg 1080

tgtaaattat ttttggaatt gagttcatag ctcgag 1116

<210> 4
<211> 1095
<212> DNA
<213> Artificial

<220>
<223> Description of artificial sequence: XhoI fragement of genetic
      construct Thy1-mmTaar1 comprising mmTaar1 cDNA fused with a
      N-terminal FLAG tag.

<400> 4
ctcgagtcca ccatgaagac gatcatcgcc ctgagctaca tcttctgcct ggtgttcgcc 60

gactacaagg acgatgatga cgccatgggc atgcatcttt gccacgctat cacaaacatt 120

tcccacagaa acagcgactg gtcaagagaa gtccaagctt ccctgtacag cttaatgtca 180

ctcataatcc tggccactct ggttggcaac ttaatagtaa taatttccat atcccatttc 240

aagcaacttc atacacccac caactggctc cttcactcca tggccattgt cgactttctg 300

ctgggctgtc tgataatgcc ctgcagcatg gtgagaactg ttgagcgctg ttggtatttt 360

ggggaaatcc tctgtaaagt tcacaccagc accgatatca tgctgagctc cgcctccatt 420

ttccacttag ctttcatttc cattgaccgc tactgtgctg tgtgtgaccc tttgagatac 480

aaagccaaga tcaatatctc cactattctt gtgatgatcc tcgttagttg gagccttcct 540

gctgtttatg catttgggat gatcttcctg gaactgaact taaaaggagt ggaagagctg 600

tatcgcagtc aggtcagcga cctgggcggc tgttctccct tctttagtaa agtatctggg 660

gtactggcgt tcatgacttc cttctatata cctggatctg ttatgttatt tgtttactat 720

aggatatatt tcatagctaa aggacaagca aggtcaatca atcgtacgaa tgttcaagtt 780

ggattggaag ggaaaagcca agcaccacaa agcaaggaaa caaaagccgc gaagacctta 840

gggatcatgg tgggcgtttt cctcgtatgc tggtgcccgt tctttctctg cacggtcctg 900

gaccctttcc tgggctatgt tatcccaccc tctctgaatg acgcactgta ttggtttggg 960

tacttgaatt ctgccctcaa tccgatggtt tatgcctttt tctatccctg gttcagaaga 1020

gccttgaaga tggttctcct tggtaaaatt ttccaaaaag attcatctag gtctaagcta 1080

tttttgtaac tcgag 1095

<210> 5
<211> 410
<212> DNA
<213> Artificial

<220>
<223> Description of artificial sequence: Sequence resulting from
      sequencing the vector construct pCMV-hsTAAR1 with T3

<400> 5
ctcgagtcca ccatgaagac gatcatcgcc ctgagctaca tcttctgcct ggtgttcgcc 60

gactacaagg acgatgatga cgccatgggc atgatgccct tttgccacaa tataattaat 120

atttcctgtg tgaaaaacaa ctggtcaaat gatgtccgtg cttccctgta cagtttaatg 180

gtgctcataa ttctgaccac actcgttggc aatctgatag ttattgtttc tatatcacac 240

ttcaaacaac ttcatacccc aacaaattgg ctcattcatt ccatggccac tgtggacttt 300

cttctggggt gtctggtcat gccttacagt atggtgagat ctgctgagca ctgttggtat 360

tttggagaag tcttctgtaa aattcacaca agcaccgaca ttatgctgag 410

<210> 6
<211> 345
<212> DNA
<213> Artificial

<220>
<223> Description of artificial sequence: sequence resulting from
      sequencing of pCMV-hsTAAR1 with T7

<400> 6
aatcagaagc tccaaattgg attggaaatg aaaaatggaa tttcacaaag caaagaaagg 60

aaagctgtga agacattggg gattgtgatg ggagttttcc taatatgctg gtgccctttc 120

tttatctgta cagtcatgga cccttttctt cactacatta ttccacctac tttgaatgat 180

gtattgattt ggtttggcta cttgaactct acatttaatc caatggttta tgcatttttc 240

tatccttggt ttagaaaagc actgaagatg atgctgtttg gtaaaatttt ccaaaaagat 300

tcatccaggt gtaaattatt tttggaattg agttcatagc tcgag 345

<210> 7
<211> 490
<212> DNA
<213> Artificial

<220>
<223> Description of artificial sequence: sequence resulting from
      sequencing of pCMV-hsTAAR1 with hu-Tar 303c.

<400> 7
ctgttggtat tttggagaag tcttctgtaa aattcacaca agcaccgaca ttatgctgag 60

ctcagcctcc attttccatt tgtctttcat ctccattgac cgctactatg ctgtgtgtga 120

tccactgaga tataaagcca agatgaatat cttggttatt tgtgtgatga tcttcattag 180

ttggagtgtc cctgctgttt ttgcatttgg aatgatcttt ctggagctaa acttcaaagg 240

cgctgaagag atatattaca aacatgttca ctgcagagga ggttgctctg tcttctttag 300

caaaatatct ggggtactga cctttatgac ttctttttat atacctggat ctattatgtt 360

atgtgtctat tacagaatat atcttatcgc taaagaacag gcaagattaa ttagtgatgc 420

caatcagaag ctccaaattg gattggaaat gaaaaatgga atttcacaaa gcaaagaaag 480

gaaagctgtg 490

<210> 8
<211> 358
<212> DNA
<213> Artificial

<220>

<223>  Description of artificial sequence: sequence resulting from
       sequencing of pCMV-hsTAAR1 with hu-Tar 408nc.

<400>  8
ctcgagtcca ccatgaagac gatcatcgcc ctgagctaca tcttctgcct ggtgttcgcc      60

gactacaagg acgatgatga cgccatgggc atgatgccct tttgccacaa tataattaat     120

atttcctgtg tgaaaaacaa ctggtcaaat gatgtccgtg cttccctgta cagtttaatg     180

gtgctcataa ttctgaccac actcgttggc aatctgatag ttattgtttc tatatcacac     240

ttcaaacaac ttcatacccc aacaaattgg ctcattcatt ccatggccac tgtggacttt     300

cttctggggt gtctggtcat gccttacagt atggtgagat ctgctgagca ctgttggt       358

<210>  9
<211>  370
<212>  DNA
<213>  Artificial

<220>
<223>  Description of artificial sequence: sequence resulting from
       sequencing of pCMV-hsTAAR1 with hu-Tar 695c.

<400>  9
acaggcaaga ttaattagtg atgccaatca gaagctccaa attggattgg aaatgaaaaa      60

tggaatttca caaagcaaag aaaggaaagc tgtgaagaca ttggggattg tgatgggagt     120

tttcctaata tgctggtgcc ctttctttat ctgtacagtc atggaccctt ttcttcacta     180

cattattcca cctactttga atgatgtatt gatttggttt ggctacttga actctacatt     240

taatccaatg gtttatgcat ttttctatcc ttggtttaga aaagcactga agatgatgct      300

gtttggtaaa attttccaaa aagattcatc caggtgtaaa ttatttttgg aattgagttc     360

atagctcgag                                                            370

<210>  10
<211>  386
<212>  DNA
<213>  Artificial

<220>
<223>  Description of artificial sequence: sequence resulting from
       sequencing of pCMV-hsTAAR1 with hu-Tar 757nc.

<400>  10
acagtatggt gagatctgct gagcactgtt ggtattttgg agaagtcttc tgtaaaattc      60

acacaagcac cgacattatg ctgagctcag cctccatttt ccatttgtct ttcatctcca     120

ttgaccgcta ctatgctgtg tgtgatccac tgagatataa agccaagatg aatatcttgg     180

ttatttgtgt gatgatcttc attagttgga gtgtccctgc tgtttttgca tttggaatga     240

tctttctgga gctaaacttc aaaggcgctg aagagatata ttacaaacat gttcactgca     300

gaggaggttg ctctgtcttc tttagcaaaa tatctggggt actgaccttt atgacttctt     360

tttatatacc tggatctatt atgtta                                          386

<210>  11
<211>  411
<212>  DNA

<213>  Artificial

<220>
<223>  Description of artificial sequence: sequence resulting from
      sequencing of pCMV-mmTaar1 with T3.

<400>  11
ctcgagtcca ccatgaagac gatcatcgcc ctgagctaca tcttctgcct ggtgttcgcc      60

gactacaagg acgatgatga cgccatgggc atgcatcttt gccacgctat cacaaacatt     120

tcccacagaa acagcgactg gtcaagagaa gtccaagctt ccctgtacag cttaatgtca     180

ctcataatcc tggccactct ggttggcaac ttaatagtaa taatttccat atcccatttc     240

aagcaacttc atacacccac caactggctc cttcactcca tggccattgt cgactttctg     300

ctgggctgtc tgataatgcc ctgcagcatg gtgagaactg ttgagcgctg ttggtatttt     360

ggggaaatcc tctgtaaagt tcacaccagc accgatatca tgctgagctc c              411

<210>  12
<211>  378
<212>  DNA
<213>  Artificial

<220>
<223>  Description of artificial sequence: sequence resulting from
      sequencing of pCMV-mmTaar1 with T7.

<400>  12
tataggatat atttcatagc taaaggacaa gcaaggtcaa tcaatcgtac gaatgttcaa      60

gttggattgg aagggaaaag ccaagcacca caaagcaagg aaacaaaagc cgcgaagacc     120

ttagggatca tggtgggcgt tttcctcgta tgctggtgcc cgttctttct ctgcacggtc     180

ctggaccctt tcctgggcta tgttatccca ccctctctga atgacgcact gtattggttt     240

gggtacttga attctgccct caatccgatg gtttatgcct ttttctatcc ctggttcaga     300

agagccttga agatggttct ccttggtaaa attttccaaa aagattcatc taggtctaag     360

ctatttttgt aactcgag                                                   378

<210>  13
<211>  371
<212>  DNA
<213>  Artificial

<220>
<223>  Description of artificial sequence: sequence resulting from
      sequencing of pCMV-mmTaar1 with mTar-293c.

<400>  13
gtgagaactg ttgagcgctg ttggtatttt ggggaaatcc tctgtaaagt tcacaccagc      60

accgatatca tgctgagctc cgcctccatt ttccacttag ctttcatttc cattgaccgc     120

tactgtgctg tgtgtgaccc tttgagatac aaagccaaga tcaatatctc cactattctt     180

gtgatgatcc tcgttagttg gagccttcct gctgtttatg catttgggat gatcttcctg     240

gaactgaact taaaaggagt ggaagagctg tatcgcagtc aggtcagcga cctgggcggc     300

tgttctccct tctttagtaa agtatctggg gtactggcgt tcatgacttc cttctatata     360

cctggatctg t                                                          371

<210> 14
<211> 292
<212> DNA
<213> Artificial

<220>
<223> Description of artificial sequence: sequence resulting from
      sequencing of pCMV-mmTaar1 with mTar-345nc.

<400> 14
ctcgagtcca ccatgaagac gatcatcgcc ctgagctaca tcttctgcct ggtgttcgcc      60

gactacaagg acgatgatga cgccatgggc atgcatcttt gccacgctat cacaaacatt     120

tcccacagaa acagcgactg gtcaagagaa gtccaagctt ccctgtacag cttaatgtca     180

ctcataatcc tggccactct ggttggcaac ttaatagtaa taatttccat atcccatttc     240

aagcaacttc atacacccac caactggctc cttcactcca tggccattgt cg             292

<210> 15
<211> 391
<212> DNA
<213> Artificial

<220>
<223> Description of artificial sequence: sequence resulting from
      sequencing of pCMV-mmTaar1 with T7.

<400> 15
gttatttgtt tactatagga tatatttcat agctaaagga caagcaaggt caatcaatcg      60

tacgaatgtt caagttggat tggaagggaa aagccaagca ccacaaagca aggaaacaaa     120

agccgcgaag accttaggga tcatggtggg cgttttcctc gtatgctggt gcccgttctt     180

tctctgcacg gtcctggacc ctttcctggg ctatgttatc ccaccctctc tgaatgacgc     240

actgtattgg tttgggtact tgaattctgc cctcaatccg atggtttatg cctttttcta     300

tccctggttc agaagagcct tgaagatggt tctccttggt aaaattttcc aaaaagattc     360

atctaggtct aagctatttt tgtaactcga g                                    391

<210> 16
<211> 451
<212> DNA
<213> Artificial

<220>
<223> Description of artificial sequence: sequence resulting from
      sequencing of pCMV-mmTaar1 with mTar-755nc.

<400> 16
cccaccaact ggctccttca ctccatggcc attgtcgact ttctgctggg ctgtctgata      60

atgccctgca gcatggtgag aactgttgag cgctgttggt attttgggga aatcctctgt     120

aaagttcaca ccagcaccga tatcatgctg agctccgcct ccattttcca cttagctttc     180

atttccattg accgctactg tgctgtgtgt gaccctttga gatacaaagc caagatcaat     240

atctccacta ttcttgtgat gatcctcgtt agttggagcc ttcctgctgt ttatgcattt     300

gggatgatct tcctggaact gaacttaaaa ggagtggaag agctgtatcg cagtcaggtc     360

agcgacctgg gcggctgttc tcccttcttt agtaaagtat ctggggtact ggcgttcatg 420

acttccttct atatacctgg atctgttatg t 451

<210> 17
<211> 21
<212> DNA
<213> Artificial

<220>
<223> Description of artificial sequence: oligonucleotide

<400> 17
cttctggggt gtctggtcat g 21

<210> 18
<211> 22
<212> DNA
<213> Artificial

<220>
<223> Description of artificial sequence: oligonucleotide

<400> 18
gcataatgtc ggtgcttgtg tg 22

<210> 19
<211> 24
<212> DNA
<213> Artificial

<220>
<223> Description of artificial sequence: oligonucleotide

<400> 19
cctggatcta ttatgttatg tgtc 24

<210> 20
<211> 23
<212> DNA
<213> Artificial

<220>
<223> Description of artificial sequence: oligonucleotide

<400> 20
cttgcctgtt ctttagcgat aag 23

<210> 21
<211> 112
<212> DNA
<213> Artificial

<220>
<223> Description of artificial sequence: oligonucleotide

<400> 21
gcctcgagtc caccatgaag acgatcatcg ccctgagcta catcttctgc ctggtgttcg 60

ccgactacaa ggacgatgat gacgccatgg gcatgatgcc cttttgccac aa 112

<210> 22
<211> 28
<212> DNA

<213>  Artificial

<220>
<223>  Description of artificial sequence: oligonucleotide

<400>  22
gcctcgagct atgaactcaa ttccaaaa                                          28


<210>  23
<211>  20
<212>  DNA
<213>  Artificial

<220>
<223>  Description of artificial sequence: oligonucleotide

<400>  23
cgactttctg ctgggctgtc                                                   20


<210>  24
<211>  21
<212>  DNA
<213>  Artificial

<220>
<223>  Description of artificial sequence: oligonucleotide

<400>  24
caacagttct caccatgctg c                                                 21


<210>  25
<211>  21
<212>  DNA
<213>  Artificial

<220>
<223>  Description of artificial sequence: oligonucleotide

<400>  25
gtactggcgt tcatgacttc c                                                 21


<210>  26
<211>  22
<212>  DNA
<213>  Artificial

<220>
<223>  Description of artificial sequence: oligonucleotide

<400>  26
ccttgcttgt cctttagcta tg                                                22


<210>  27
<211>  115
<212>  DNA
<213>  Artificial

<220>
<223>  Description of artificial sequence: oligonucleotide

<400>  27
gcctcgagtc caccatgaag acgatcatcg ccctgagcta catcttctgc ctggtgttcg      60

ccgactacaa ggacgatgat gacgccatgg gcatgcatct ttgccacgct atcac          115

<210>  28
<211>  28
<212>  DNA
<213>  Artificial

<220>
<223>  Description of artificial sequence: oligonucleotide

<400>  28
gcctcgagtt acaaaaatag cttagacc                                          28


<210>  29
<211>  20
<212>  DNA
<213>  Artificial

<220>
<223>  Description of artificial sequence: oligonucleotide

<400>  29
aattaaccct cactaaaggg                                                   20


<210>  30
<211>  20
<212>  DNA
<213>  Artificial

<220>
<223>  Description of artificial sequence: oligonucleotide

<400>  30
taatacgact cactataggg                                                   20


<210>  31
<211>  20
<212>  DNA
<213>  Artificial

<220>
<223>  Description of artificial sequence: oligonucleotide

<400>  31
gtgctccgtg gatctcaagc                                                   20


<210>  32
<211>  21
<212>  DNA
<213>  Artificial

<220>
<223>  Description of artificial sequence: oligonucleotide

<400>  32
ggatgatggc atgcagcact g                                                 21


<210>  33
<211>  18
<212>  DNA
<213>  Artificial

<220>
<223>  Description of artificial sequence: forward primer

<400>  33

gatcatcgcc ctgagcta 18

<210> 34
<211> 20
<212> DNA
<213> Artificial

<220>
<223> Description of artificial sequence: Reverse primer

<400> 34
gcgtcatcat cgtccttgta 20

<210> 35
<211> 16
<212> DNA
<213> Artificial

<220>
<223> Dcscription of artificial scqucncc: FAM labclcd probc

<400> 35
tcttctgcct ggtgtt 16